(19) Europäisches Patentamt
European Patent Office
Office européen des brevets

(11) **EP 2 397 160 A1**

(12) **EUROPEAN PATENT APPLICATION**
published in accordance with Art. 153(4) EPC

(43) Date of publication:
**21.12.2011 Bulletin 2011/51**

(51) Int Cl.:
*A61K 47/32* (2006.01)   *A61K 9/48* (2006.01)
*A61K 47/08* (2006.01)   *A61K 47/10* (2006.01)
*A61K 47/12* (2006.01)   *A61K 47/14* (2006.01)
*A61K 47/34* (2006.01)

(21) Application number: **10741297.5**

(22) Date of filing: **12.02.2010**

(86) International application number:
**PCT/JP2010/052091**

(87) International publication number:
**WO 2010/093020 (19.08.2010 Gazette 2010/33)**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **13.02.2009 JP 2009031370**

(71) Applicant: **Nisshin Kasei Co., Ltd.
Osaka 541-0045 (JP)**

(72) Inventors:
• **MORIUCHI, Toshiaki
Yamatokoriyama-shi
Nara 639-1058 (JP)**

• **TAGUCHI, Makoto
Kadoma-shi
Osaka 571-0078 (JP)**
• **KOJO, Akane
Kyoto-shi
Kyoto 603-8212 (JP)**
• **YOSHINO, Hiroyuki
Kobe-shi
Hyogo 651-1513 (JP)**

(74) Representative: **Müller-Boré & Partner
Patentanwälte
Grafinger Straße 2
81671 München (DE)**

(54) **HARD CAPSULE**

(57)   The object of the invention is to provide a hard capsule that is excellent in stability even when filled with a solvent for dissolving a poorly soluble pharmaceutical active ingredient, and that has excellent mechanical strength in a low-humidity environment. The invention provides a hard capsule having a film containing (A) a polymer or copolymer obtained by polymerizing or copolymerizing at least one polymerizable vinyl monomer represented by Formula (1): $H_2C = C(R_1)\text{-}COOR_2$ (1), wherein $R_1$ represents hydrogen or methyl, and $R_2$ represents hydrogen or $C_1\text{-}C_4$ alkyl, in the presence of polyvinyl alcohol and/or a derivative thereof; and (B) at least one compound selected from the group consisting of (B-1) polyhydric alcohols, (B-2) esters of polyhydric alcohols and $C_1\text{-}C_5$ carboxylic acids, and (B-3) esters of polyvalent carboxylic acids and $C_1\text{-}C_5$ alcohols. The capsule of the present invention has excellent mechanical strength, while maintaining low moisture content, and thus exhibits excellent physical stability even when filled with a poorly soluble drug-dissolving solvent.

EP 2 397 160 A1

## Description

Technical Field

The present invention relates to a hard capsule comprising a film that comprises a polymer or copolymer obtained by polymerizing or copolymerizing at least one polymerizable vinyl monomer in the presence of polyvinyl alcohol and/or a derivative thereof, and a specific compound.

Background Art

A large number of the active substances of medicines, i.e., pharmaceutical active ingredients, are poor in water solubility. Such substances are poorly absorbed from the alimentary tract, and the bioavailability and drug efficacy expression are thus easily reduced or are subject to fluctuation. In preclinical tests, to evaluate drug efficacy or obtain biopharmaceutical parameters using animals or the like, it is common to dissolve the pharmaceutical active ingredient in some solvent to make it more easily absorbed. For a poorly soluble pharmaceutical active ingredient, a polyethylene glycol having relatively low molecular weight and a derivative thereof, a polyoxyethylene sorbitan fatty acid ester, a fatty acid having 6 to 12 carbon atoms or a salt thereof, polyoxyethylene castor oil, a diethylene glycol derivative, or the like may be used. However, these solvents are usually in liquid form, and forming them into tablets is difficult. Therefore, additional consideration must be given to the ultimate dosage form for sale in the market. If these solvents could be directly formulated into pharmaceutical preparations, the time required for the formulation could be greatly shortened. A capsule is highly anticipated to serve as such a dosage form.

Capsules hitherto known are those produced using gelatin or a cellulose derivative as a base material. When a known gelatin hard capsule is filled with a polyethylene glycol having a weight average molecular weight of 400 (PEG 400), the moisture in the capsule film migrates into the solvent, causing the capsule to break (see Non-Patent Literature (NPL) 1). In known cellulose derivative-based capsules, the aforementioned solvents act as plasticizers, causing them to, for example, permeate the capsule film and be exuded to the capsule surface.

In order to solve such problems, a hard capsule that is made mainly of a polymer or copolymer obtained by polymerizing or copolymerizing at least one polymerizable vinyl monomer in the presence of polyvinyl alcohol and/or a derivative thereof has been reported (see Patent Literature (PTL) 1).

Citation List

Patent Literature

[0005]

　　PTL 1: WO 2002/017848

Non-Patent Literature

[0006]

　　NPL 1: Pharmaceutical Technology Europe, October, 84, 86, 88-90, 1998

Summary of Invention

Technical Problem

To date, a hard capsule that mainly comprises a polymer or copolymer obtained by polymerizing or copolymerizing at least one polymerizable vinyl monomer in the presence of polyvinyl alcohol and/or a derivative thereof has not exhibited sufficient mechanical strength when stored in a low-humidity environment.

The primary object of the present invention is to provide a hard capsule that is excellent in stability even when filled with a solvent for dissolving a poorly soluble pharmaceutical active ingredient (hereinafter sometimes referred to as a "poorly soluble drug-dissolving solvent"), and that has excellent mechanical strength in a low-humidity environment.

Solution to Problem

The present inventors conducted extensive research to achieve the above object and found the following. A hard capsule produced by using a specific compound, and a polymer or copolymer obtained by polymerizing or copolymerizing at least one polymerizable vinyl monomer in the presence of polyvinyl alcohol and/or a derivative thereof is excellent in stability even when filled with a solvent for dissolving a poorly soluble pharmaceutical active ingredient, and has excellent general characteristics that hard capsules generally have, such as water solubility. The present inventors further found that the film of such a hard capsule is excellent in mechanical strength in a low-humidity environment. The inventors conducted further research, and accomplished the present invention.

More specifically, the present invention provides the following hard capsule, etc.

Item 1.

A hard capsule having a film comprising:

(A) a polymer or copolymer obtained by polymerizing or copolymerizing at least one polymerizable vinyl monomer represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms, in the presence of polyvinyl alcohol and/or a derivative thereof; and

(B) at least one compound selected from the group consisting of (B-1) polyhydric alcohols, (B-2) esters of polyhydric alcohols and carboxylic acids having 1 to 5 carbon atoms, and (B-3) esters of polyvalent carboxylic acids and alcohols having 1 to 5 carbon atoms.

Item 2.

The hard capsule according to Item 1, wherein, in the compound of (B), the polyhydric alcohol is selected from the group consisting of sorbitol, mannitol, glycerol, and propylene glycol; and the polyvalent carboxylic acid is citric acid.

Item 3.

The hard capsule according to Item 1 or 2, wherein the compound of (B) is at least one member selected from the group consisting of glycerol, propylene glycol, triacetin, and triethyl citrate.

Item 4.

The hard capsule according to any of Items 1 to 3, comprising the compound of (B) in an amount of 0.1 to 2 parts by weight, relative to 10 parts by weight of the polymer or copolymer of (A).

Item 5.

The hard capsule according to any of Items 1 to 4, which is to be filled with at least one member selected from the group consisting of

(a) polyethylene glycols having a weight average molecular weight of 2,000 or less, or derivatives thereof,
(b) polyoxyethylene sorbitan fatty acid esters,
(c) fatty acids having 6 to 12 carbon atoms or salts thereof,
(d) polyoxyethylene castor oil,
(e) diethylene glycol ether derivatives,
(f) aliphatic alcohols having 6 to 12 carbon atoms, and
(g) polyoxyethylene sorbitol fatty acid esters.

Item 6.

The hard capsule according to any of Items 1 to 5, wherein the film further comprises (C) a gelling agent.

Item 7.

The hard capsule according to any of Items 1 to 6, wherein the polymer or copolymer of (A) is a polymer or copolymer obtained by polymerizing or copolymerizing at least one polymerizable vinyl monomer in the presence of polyvinyl alcohol.

Item 8.

The hard capsule according to any of Items 1 to 6, wherein the polyvinyl alcohol derivative in (A) possesses a thiol group at a terminus.

Item 9.

The hard capsule according to any of Items 1 to 8, wherein the polymerizable vinyl monomer in (A) comprises acrylic acid or methacrylic acid and methyl methacrylate, and wherein the acrylic acid or methacrylic acid is contained in an amount of 5 to 50 wt%, and the methyl methacrylate is contained in an amount of 50 to 95 wt%, based on the total amount of the polymerizable vinyl monomer.

Item 10.

The hard capsule according to any of Items 1 to 9, wherein the polyvinyl alcohol and/or derivative thereof in (A) is used in an amount of 20 to 95 wt%, and the polymerizable vinyl monomer is used in an amount of 5 to 80 wt%.

Item 11.

The hard capsule according to any of Items 1 to 10, which is filled with at least one member selected from the group consisting of

(a) polyethylene glycols having a weight average molecular weight of 2,000 or less, or derivatives thereof,
(b) polyoxyethylene sorbitan fatty acid esters,
(c) fatty acids having 6 to 12 carbon atoms or salts thereof,
(d) polyoxyethylene castor oil,
(e) diethylene glycol ether derivatives,
(f) aliphatic alcohols having 6 to 12 carbon atoms, and
(g) polyoxyethylene sorbitol fatty acid esters.

Item 12.

A method for producing a film of the hard capsule of any of Items 1 to 10, comprising:

immersing a capsule molding pin in an aqueous solution containing (A) a polymer or copolymer obtained by polymerizing or copolymerizing at least one polymerizable vinyl monomer represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms, in the presence of polyvinyl alcohol and/or a derivative thereof, and (B) at least one compound selected from the group consisting of (B-1) polyhydric alcohols, (B-2) esters of polyhydric alcohols and carboxylic acids having 1 to 5 carbon atoms, and (B-3) esters of polyvalent carboxylic acids and alcohols having 1 to 5 carbon atoms; withdrawing the immersed capsule molding pin from the solution; and drying the aqueous solution adhering to the pin.

Item 13.

Use of (A) a polymer or copolymer obtained by polymerizing or copolymerizing at least one polymerizable vinyl monomer represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms, in the presence of polyvinyl alcohol and/or a derivative thereof, and (B) at least one compound selected from the group consisting of (B-1) polyhydric alcohols, (B-2) esters of polyhydric alcohols and carboxylic acids having 1 to 5 carbon atoms, and (B-3) esters of polyvalent carboxylic acids and alcohols having 1 to 5 carbon atoms, in producing a hard capsule.

Item 14.

The hard capsule according to Item 11, which is further filled with a thickener.

Advantageous Effects of Invention

The hard capsule of the present invention is excellent in stability even when filled with a poorly soluble drug-dissolving solvent, and in mechanical strength in a low-humidity environment.

Specifically, the present invention provides a hard capsule that can be filled with various types of pharmaceutical active ingredients that have been considered unsuitable for filling a capsule therewith, and that is excellent in mechanical strength during storage. This contributes to, for example, the practical utilization of various types of drugs, and an improvement in capsule quality.

Brief Description of Drawings

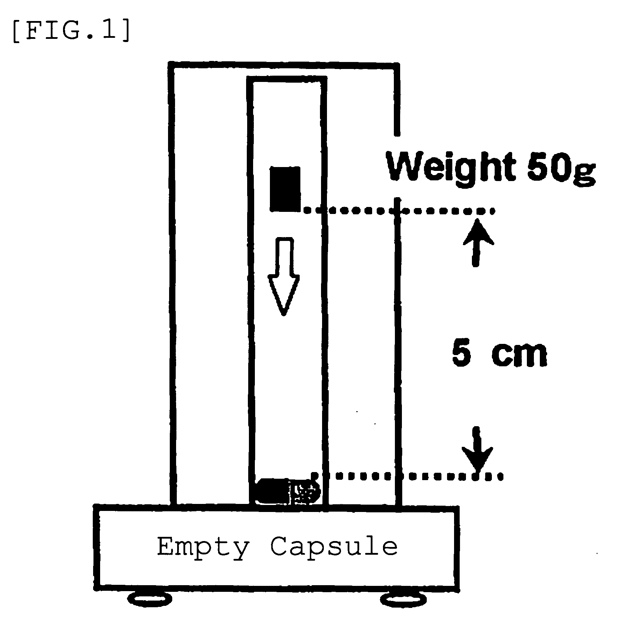

Figure 1 schematically illustrates an impact strength testing machine for hard capsules.

Description of Embodiments

Hereinafter, the present invention is described in more detail.

1. Film

It is essential that the film of the hard capsule of the present invention comprises (A) a polymer or copolymer obtained by polymerizing or copolymerizing at least one polymerizable vinyl monomer represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms, in the presence of polyvinyl alcohol and/or a derivative thereof, and (B) at least one compound selected from the group consisting of (B-1) polyhydric alcohols, (B-2) esters of polyhydric alcohols and carboxylic acids having 1 to 5 carbon atoms, and (B-3) esters of polyvalent carboxylic acids and alcohols having 1 to 5 carbon atoms.

(A) Polymer or copolymer obtained by polymerizing or copolymerizing at least one polymerizable vinyl monomer in the presence of polyvinyl alcohol and/or a derivative thereof

Polyvinyl alcohols (sometimes referred to as PVA) and derivatives thereof usable in the present invention are completely saponified PVA, intermediately saponified PVA, partially saponified PVA, as well as various modified PVAs, such as amine-modified PVA, ethylene-modified PVA, terminal-thiol-modified PVA, and the like.

PVAs can be obtained by radical-polymerizing vinyl acetate, and suitably saponifying the obtained vinyl acetate. Therefore, PVAs generally have $-OCOCH_3$ groups originating from vinyl acetate. PVAs can be classified into those that are completely saponified, intermediately saponified, partially saponified, and the like, depending on the degree of saponification. PVAs that are usable in the present invention preferably have a saponification degree of about 70 mol% or more, more preferably about 80 mol% or more, and still more preferably 85 mol% or more. Of these, saponificated PVAs with a saponification degree of 85 to 90 mol%, and in particular about 86 to 89 mol%, are preferable. As is well known in this field, completely saponified PVA generally refers to PVA with a saponification degree of 98 mol% or more, and does not necessarily indicate PVA with a saponification degree of 100 mol%.

Examples of PVA derivatives include various kinds of modified PVAs, such as amine-modified PVA, ethylene-modified PVA, and terminal-thiol-modified PVA. These modified PVAs may be produced by, for example, methods known in this field.

Commercially available PVAs and derivatives thereof may also be used. They may be purchased from, for example, Nippon Synthetic Chemical Industry Co., Ltd., Japan Vam &. Poval Co., Ltd., or the like.

PVAs are known to have various polymerization degrees. The average polymerization degree is not limited, as long as a PVA that is optimum in terms of concentration and viscosity is selected in accordance with its usage. Specifically, there are various methods for producing a hard capsule, as shown, for example, in "Item 2. Production Method" below, and the optimum viscosity therefor depends on each of the methods. The molecular weight of PVAs applicable therein can also be suitably selected.

As one embodiment, PVAs usable in the present invention are those having a weight average molecular weight of about 30,000 to 400,000, and preferably about 100,000 to 300,000. The weight average molecular weight of PVA is a value measured by a GPC method (nonaqueous size exclusion chromatography). Specifically, the weight average molecular weight is measured as follows: PVA is dissolved in dimethyl sulfoxide (DMSO) containing lithium chloride at a concentration of 10 moL so that the concentration of the PVA is 1 mg/mL; the mixture is stirred while heating at 40°C for 30 minutes, and then left to stand at room temperature overnight; the resulting product is filtrated through a PTFE cartridge filter (0.45 μm), followed by measurement of the molecular weight distribution by a GPC method.

Further, PVAs usable in the present invention are those having an average polymerization degree of, for example, about 350 to 5,000, and preferably about 1,200 to 3,800.

PVAs and derivatives thereof may be used singly or in a combination of two or more. For example, PVAs having different degrees of saponification and various modified PVAs may be used singly or in a combination of two or more. Commercially available PVAs and derivatives thereof can also be used.

Polymerizable vinyl monomers usable in the present invention are compounds represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms.

Specific examples of the polymerizable vinyl monomers usable in the present invention include acrylic acid, methacrylic acid, methyl methacrylate, methyl acrylate, ethyl methacrylate, ethyl acrylate, butyl methacrylate, butyl acrylate, isobutyl methacrylate, and isobutyl acrylate. Salts of acrylic acid or methacrylic acid can also be used. Examples of such salts include sodium salt, potassium salt, ammonium salt, and alkylamine salt.

The polymerizable vinyl monomers may be used singly or in a combination of two or more.

As the polymerizable vinyl monomers, it is preferable to use at least one of acrylic acid and methacrylic acid, in combination with at least one member selected from the group consisting of methyl methacrylate, methyl acrylate, ethyl methacrylate, ethyl acrylate, butyl methacrylate, butyl acrylate, isobutyl methacrylate, and isobutyl acrylate. It is more preferable to use acrylic acid or methacrylic acid, in combination with methyl methacrylate.

In a PVA copolymer, the weight ratio of the PVA and/or derivative thereof to the polymerizable vinyl monomer is not particularly limited. However, it is preferable that the PVA and/or derivative thereof be used in an amount of 20 to 95 wt%, and the polymerizable vinyl monomer be used in an amount of 5 to 80 wt%. It is more preferable that the PVA and/or derivative thereof be used in an amount of 50 to 90 wt%, and the polymerizable vinyl monomer be used in an amount of 10 to 50 wt%.

It is preferable that the PVA and/or derivative thereof be used in an amount of 20 wt% or greater, rather than less than 20 wt%, because when the amount of the PVA and/or derivative thereof is 20 wt% or greater, the produced capsule shows more improved dissolution or dispersion ability in water. In addition, when the amount of the PVA and/or derivative thereof is 95 wt% or less, the produced capsule is less easily affected by humidity so that the capsule is not easily weakened in strength under high humidity, compared with the case where the amount of the PVA and/or derivative thereof exceeds 95 wt%.

When two or more polymerizable vinyl monomers are used in combination, the ratio is not particularly limited. However, when at least one member selected from the group (I) consisting of acrylic acid and methacrylic acid; and sodium salts, potassium salts, ammonium salts, and alkylamine salts, of acrylic acid and methacrylic acid is used in combination with at least one member selected from the group (II) consisting of methyl methacrylate, methyl acrylate, ethyl methacrylate, ethyl acrylate, butyl methacrylate, butyl acrylate, isobutyl methacrylate, and isobutyl acrylate, the weight ratio thereof is as follows: the at least one member selected from the group (I) is used in an amount of 5 to 50 wt%, and preferably 10 to 40 wt%, and the at least one member selected from the group (II) is used in an amount of 50 to 95 wt%, and preferably 60 to 90 wt%, based on the total amount of the polymerizable vinyl monomers.

A known method may be used for the copolymerization. For example, the PVA and/or derivative thereof is added to water, and the mixture is heated to effect dissolution. Then, at least one polymerizable vinyl monomer and a polymerization initiator are added thereto to initiate the copolymerization, thereby obtaining a resin. The weight ratio of the PVA and/or derivative thereof to the polymerizable vinyl monomer in the PVA copolymer is determined according to the weight ratio of those added to water, i.e., the PVA and/or derivative thereof to the polymerizable vinyl monomer. Therefore, when added to water, the weight ratio of the PVA and/or derivative thereof to polymerizable vinyl monomer is preferably equal to the above-mentioned weight ratio in the PVA copolymer.

Usable polymerization initiators are those hitherto used. Examples thereof include 2,2'-azobis(2-amidinopropane) hydrochloride, AIBN (azoisobutyronitrile), and like azo compounds; potassium persulfate, sodium persulfate, ammonium persulfate, and like persulfates; t-butyl hydroperoxide and like organic peroxides; and hydrogen peroxide-tartaric acid, hydrogen peroxide-sodium tartrate, and like redox initiators.

The amount of the PVA copolymer of (A) is preferably 80 to 98 wt% (dry weight), relative to the total amount of the film.

According to the present invention, although restrictive interpretation is not intended, the reaction mechanism of the polymerization or copolymerization of at least one specific polymerizable vinyl monomer in the presence of polyvinyl alcohol and/or a derivative thereof is assumed to be as follows: first, a polymerization initiator abstracts hydrogen from the methyl group at the terminal of -OCOCH$_3$ present in the PVA, creating a radical. Then, the polymerizable vinyl monomer bonds to the radical, allowing the double bond of the polymerizable vinyl monomer to be cleaved, thereby again creating a radical. Then, the polymerizable vinyl monomer bonds to the radical; the reaction is repeated in the same manner as above.

In the present invention, the PVA copolymer of (A) has a structure in which at least one of the aforementioned polymerizable vinyl monomers is graft polymerized with -OCOCH$_3$, which is a side chain of PVA. In this graft polymerization, PVA may be joined together through a polymer obtained by polymerization or copolymerization of at least one of the polymerizable vinyl monomers.

For example, when acrylic acid and methyl methacrylate are used as polymerizable vinyl monomers, the PVA copolymer of (A) has a structure in which a copolymer of acrylic acid and methyl methacrylate is bonded to PVA through -OCOCH$_3$ of the PVA. Specific examples of such PVA copolymers (copolymers of polyvinyl alcohol/acrylic acid/methyl methacrylate) include POVACOAT® Type R and POVACOAT® Type L (produced by Daido Chemical Corporation), which are used in the Examples described below.

<u>(B) At least one compound selected from the group consisting of (B-1) polyhydric alcohols, (B-2) esters of polyhydric alcohols and carboxylic acids having 1 to 5 carbon atoms, and (B-3) esters of polyvalent carboxylic acids and alcohols having 1 to 5 carbon atoms</u>

The film of the hard capsule of the present invention further comprises the compound of (B), thereby achieving improved mechanical strength, particularly impact resistance in a relatively low-humidity environment at a relative humidity (RH) of, for example, 40% or less.

The polyhydric alcohols are not limited as long as they contain two or more hydroxyl groups. Preferable examples thereof include glycerol, ethylene glycol, diethylene glycol, triethylene glycol, polyethylene glycol, propylene glycol, polypropylene glycol, diglycerol, 1,3-butylene glycol, and sugar alcohols. Examples of sugar alcohols include sorbitol, mannitol, erythritol, and xylitol. Of these, glycerol, propylene glycol, sorbitol, and mannitol are preferable, and glycerol and propylene glycol are more preferable.

As the esters of polyhydric alcohols, an ester of a polyhydric alcohol and a carboxylic acid having 1 to 5 carbon atoms, preferably 1 to 4 carbon atoms, and more preferably 1 to 3 carbon atoms, is used. Preferable examples thereof include monoesters, diesters, triesters, etc. of the aforementioned polyhydric alcohols. Specific preferable examples of the esters of polyhydric alcohols include glycerol triacetate (hereinafter sometimes referred to as "triacetin"), glycerol monoacetate, glycerol diacetate, glycerol tributyrate, glycerol tripropionate, propylene glycol diacetate, and ethylene glycol dibutyrate. Of these, triacetin is particularly preferable.

As the esters of polyvalent carboxylic acids, an ester of a polyvalent carboxylic acid and alcohol having 1 to 5 carbon atoms, preferably 1 to 4 carbon atoms, and more preferably 1 to 3 carbon atoms, is used. Preferable examples thereof include monoesters, diesters, triesters, etc., of polyvalent carboxylic acids. The polyvalent carboxylic acids are not limited as long as they have two or more carboxyl groups. Specific preferable examples of polyvalent carboxylic acids include citric acid, acetylcitric acid, tartaric acid, malic acid, fumaric acid, maleic acid, malonic acid, glutaric acid, adipic acid, and succinic acid.

Specific preferable examples of the esters of polyvalent carboxylic acids include triethyl citrate, tributyl citrate, acetyl triethyl citrate, diethyl succinate, and dimethyl succinic acid. Of these, triethyl citrate is particularly preferable.

The (B-1) polyhydric alcohols, (B-2) esters of polyhydric alcohols and carboxylic acids having 1 to 5 carbon atoms, and (B-3) esters of polyvalent carboxylic acids and alcohols having 1 to 5 carbon atoms may be used singly or in a combination of two or more.

Among the above, as the compound of (B) of the present invention, at least one member selected from glycerol and glycerol esters, propylene glycol and propyleneglycol esters, and citric acid esters is particularly preferably used.

Of these, glycerol, propylene glycol, triacetin, and triethyl citrate are particularly preferable, because they effectively improve mechanical strength, and impart excellent stability to a capsule filled with a poorly soluble drug-dissolving solvent.

The amount of the compound of (B) is not particularly limited as long as it achieves the effects of the present invention, but it is generally about 1 to 20 wt%, preferably 2 to 15 wt%, and more preferably about 3 to 10 wt%, on a dry weight basis, relative to the total weight of the film. The use of the compound of (B) in an amount within the above-mentioned range can impart more enhanced mechanical strength to the film, and excellent formability of the capsule.

The film of the hard capsule of the present invention preferably comprises (B) in an amount of 0.1 to 2 parts by weight, more preferably 0.1 to 1 part by weight, and still more preferably 0.2 to 1 part by weight, relative to 10 parts by weight of (A). The film comprising (A) and (B) in the above-mentioned ratio range exhibits excellent mechanical strength, while maintaining the moisture content low, and thus shows excellent physical stability even when the capsule is filled with a poorly soluble drug-dissolving solvent.

(C) Other Components

The film may also comprise another component in addition to (A) and (B) above.

For example, when the gelation ability is poor, a known gelling agent may be added.

Examples of gelling agents usable in the production of a hard capsule comprising a water soluble cellulose derivative as a base include the gelling agent suggested in Japanese Patent No. 2552937.

A usable gelling agent is appropriately selected according to its compatibility with the mixture of (A) and (B). Specific examples thereof include kappa carrageenan, iota carrageenan, lambda carrageenan, tamarind seed polysaccharide, pectin, curdlan, gelatin, furcellaran, agar, xanthan gum, locust bean gum, and gellant gum. These may be used singly or in a combination of two or more.

A gelling aid may also be used, if necessary. A gelling aid may be suitably selected according to the type of the gelling agent to be used. Specifically, for example, with kappa carrageenan, a water-soluble compound containing one or more of potassium ions, ammonium ions, and calcium ions may be used. Examples thereof include potassium chloride, potassium phosphate, calcium chloride, and ammonium chloride. With iota carrageenan, a water-soluble compound containing calcium ions may be used. Examples thereof include calcium chloride.

The amount of a gelling agent is suitably determined in accordance with the type, etc., of the gelling agent, but is preferably, for example, 0.05 to 10 wt%, and more preferably 0.1 to 3 wt%, on a dry weight basis, relative to the total weight of the film. The amount of a gelling aid is also suitably determined in accordance with the type, etc., of the gelling agent, but is, for example, 0.05 to 10 wt%, and more preferably 0.1 to 3 wt%, on a dry weight basis, relative to the total weight of the film.

Similar to common hard gelatin capsules or cellulose derivative capsules, the hard capsule of the present invention may arbitrarily comprise a dye, a pigment, and like colorants; an opacifying agent; a flavor; sodium lauryl sulfate and like surfactants; and the like, within a range that does not hinder the effects of the present invention. The amount thereof added to the hard capsule is suitably adjusted within a range that enables the production of the hard capsule.

The thickness of the film of the hard capsule is not particularly limited, as long as the functions as a hard capsule are satisfactory, but is generally about 0.01 to 5 mm, preferably about 0.05 to 1 mm, and more preferably about 0.05 to 0.5 mm.

2. Production Method

The hard capsule comprising a film of the present invention may be produced by, for example, an injection molding method, a dipping method, or the like. The production method is not particularly limited to the above as long as a hard capsule can be produced, and the same methods that are used to produce general hard gelatin capsules may be used.

A dipping method produces a capsule by using the fact that a hard capsule base material turns into a gel due to a temperature difference. When the base material does not exhibit gelation ability, an aforementioned gelling agent, and further, an aforementioned gelling aid, if necessary, can also be added to produce a hard capsule.

An embodiment is given below with respect to a method for producing a hard capsule by using a gelling agent. A molding pin is immersed in an aqueous solution (a gel) containing a dissolved composition comprising (A), (B), and (C) a gelling agent, and, if necessary, (D) a gelling aid; and the immersed pin is withdrawn therefrom. The aqueous solution is cooled, if needed, to allow the solution to gel. The obtained product is then dried to form a film. Specifically, a capsule molding pin is immersed in an aqueous solution (a capsule preparation liquid) containing dissolved (A) to (D), and withdrawn therefrom. Then, the aqueous solution (the capsule preparation liquid) adhering to the molding pin is dried to form the film of a hard capsule. For example, when (C) a gelling agent and (D) a gelling aid are not used, a film may be obtained, in a manner similar to the above, by using an aqueous solution (a capsule preparation liquid) containing dissolved (A) and (B).

In the preparation of the capsule preparation liquid, there are no particular limitations to the order of dissolution of each component in the preparation liquid.

More specifically, the methods described in the Examples are exemplified.

3. Hard Capsule

The hard capsule of the present invention is sufficient if it comprises the above-described film. The hard capsule of the present invention also includes a capsule with content (an encapsulated formulation), and an empty capsule without content.

Although there are no particular limitations on the types of content filled in the capsule, the hard capsule of the present invention is preferably used for enclosing, in particular, a poorly soluble drug-dissolving solvent, or a pharmaceutical active ingredient that is considered to cause an adverse effect on stability.

Specifically, filling a known hard capsule with a poorly soluble drug-dissolving solvent would cause breakage, etc., of the capsule. However, the hard capsule of the present invention does not suffer from such a problem, and is advantageous in that it is capable of enclosing a poorly soluble pharmaceutical active ingredient. Therefore, it is also an advantage of the hard capsule of the present invention that it can be filled with a pharmaceutical active ingredient that has an adverse effect on the stability of known hard capsules. Further, the hard capsule of the present invention exhibits an excellent low moisture property, compared with known hard capsules (e.g., gelatin capsules). The term "low moisture property" refers to a property of low moisture content at an ambient humidity (e.g., 25°C, 40% RH). When the film of a hard capsule has a high moisture content, the moisture in the film migrates into the drug and solvent filled in the capsule, possibly decreasing the stability of the drug and solvent. Therefore, it is preferable that hard capsules have a low moisture property.

Poorly soluble drugs refer to those having poor water solubility, and may be any of those defined as "Sparingly soluble", "Slightly soluble", "Very slightly soluble", or "Practically insoluble or insoluble", as described in the Japanese Pharmacopoeia Fifteenth Edition. Specifically, the degree of dissolution within 30 minutes is evaluated by forming a drug into a powder when the drug is a solid, then vigorously shaking the powder in water at $20 \pm 5$°C for 30 seconds at 5-minute intervals. When the amount of water required to dissolve 1 g or 1 mL of a drug is 30 mL or more and less than 100 ml, the drug is evaluated as "Sparingly soluble"; when the amount is 100 mL or more and less than 1,000 mL, the drug is evaluated as "Slightly soluble"; when the amount is 1,000 mL or more and less than 10,000 mL, the drug is evaluated as "Very slightly soluble"; and when the amount is 10,000 mL or more, the drug is evaluated as "Practically insoluble or insoluble".

Examples of poorly soluble drug-dissolving solvents include polyethylene glycols and derivatives thereof, diethylene glycol ether derivatives, propylene glycol fatty acid esters, glycerin fatty acid esters, polyglyceryl fatty acid esters, polyoxyethylene glycerin fatty acid esters, sorbitan fatty acid esters, polyoxyethylene sorbitan fatty acid esters, polyoxyethylene sorbitol fatty acid esters, polyoxyethylene castor oil, medium chain fatty acids and salts thereof, and medium chain aliphatic alcohols.

As polyethylene glycols, those having a low molecular weight are preferable. Examples thereof include polyethylene glycols having a weight average molecular weight of 2,000 or less, preferably 1,500 or less, and more preferably 1,000 or less. Specific examples thereof include PEG 400 (a polyethylene glycol having a weight average molecular weight of about 400). Examples of the derivatives thereof include fatty acid ester derivatives. The weight average molecular weights of polyethylene glycols are values measured in the following manner. Specifically, 42 g of phthalic anhydride is added to a 1-L ground-in stopper bottle that is protected from light and that contains 300 mL of newly distilled pyridine. The resulting product is vigorously shaken to effect dissolution, and then left to stand for 16 hours or more. Thereafter, 25 mL of the obtained liquid is introduced into a pressure resistant ground-in stopper bottle (about 200 mL), followed by the addition of about 0.8 to 15 g of a PEG sample to be measured. The resulting bottle is sealed, enclosed in durable fabric, and placed in a water bath that has been heated to $98 \pm 2$°C in advance. At this time, the bottle is placed in the water bath so that the liquid in the bottle is immersed in the water bath. After the temperature is kept at $98 \pm 2$°C for 30 minutes, the bottle is taken out of the water bath, and cooled in air to room temperature. Subsequently, 50 mL of 0.5 mol/L sodium hydroxide liquid is added thereto, followed by further addition of five drops of a pyridine solution of phenolphthalein (1 → 100). The resulting liquid is titrated with 0.5 mol/L sodium hydroxide liquid, provided that the titration is terminated when the liquid shows a pale red color continuously for 15 seconds. A blank experiment is carried out in a manner similar to the above. The weight average molecular weight is calculated using the following formula:

```
Average molecular weight =

        (the amount of sample (g) x 4,000/(a-b))
```

a: the amount (mL) of 0.5 mol/L sodium hydroxide liquid consumed in the blank experiment
b: the amount (mL) of 0.5 mol/L sodium hydroxide liquid consumed in the experiment of PEG sample

Examples of medium chain fatty acids and salts thereof include fatty acids having 6 to 12 carbon atoms and salts thereof. Specific examples thereof include caproic acid, caprylic acid, capric acid, and lauric acid, and sodium salts and potassium salts of these acids.

Examples of medium chain aliphatic alcohols include aliphatic alcohols having 6 to 12 carbon atoms. Specific examples thereof include caproyl alcohol, capryl alcohol, and lauryl alcohol.

Note that the solvents that can be used to fill the hard capsule of the present invention are not limited to the above, and poorly soluble drug-dissolving solvents other than the above may also be used. It is also possible to use a mixture of the above solvents in combination with other known solvents.

Pharmaceutical modifications may also be made by, for example, adding a thickener to the solvents to simplify the filling procedure, or to prevent leakage of the filled material from the hard capsule. There are no particular limitations on thickeners, as long as they are those described in textbooks of pharmaceutics, or those generally used, such as light anhydrous silicic acid, vegetable oils, and cellulose derivatives. The amount of thickener added is preferably, for example, 0.1 to 10 parts by weight, more preferably 0.3 to 3 parts by weight, relative to 100 parts by weight of a poorly soluble drug-dissolving solvent.

The pharmaceutical active ingredients that are used to fill the hard capsule of the present invention are not particularly limited, as long as they do not impair the functions of the capsule.

As medicines, for example, the following can be used: vitamins, antifebrile, analgesics, antiphlogistics, antiulcer drugs, cardiotonics, anticoagulants, hemostatic agents, bone resorption inhibitors, vascularization inhibitors, antidepressants, antitumor agents, antitussives/expectorants, muscle relaxants, antiepileptics, antiallergic agents, antiarrhythmics, vasodilators, antihypertensive diuretics, diabetes drugs, antituberculous agents, hormonal agents, antinarcotics, antibacterials, antifungals, antivirals, and the like. Note that the medicines are not particularly limited to these pharmacological classes, and any materials that contain pharmaceutical active ingredients with relatively poor water solubility can be used to fill the hard capsule of the present invention. If a medicinal product is dissolvable in water, it is surely possible to preliminarily dissolve it in water to fill the hard capsule of the present invention therewith. A medicinal product in the form of a powder or granules may also be used as is to fill the hard capsule.

The hard capsule of the present invention can also be preferably used to enclose a poorly soluble active substance.

Filling the hard capsule with an additive, such as lactose or starch, which is used in general hard capsules, is also not limited.

Further, there is no limitation on the form of the contents to be used to fill the capsule, and it may be in the form of a liquid, a suspension, a powder, granules, a paste, a semi-solid ointment, a cream, or the like.

It is also possible to add other known capsule techniques to the hard capsule of the present invention, as required. For example, if the area where the cap and body of a capsule meet is sealed with, for example, a material similar to the film of the capsule, leakage or dissipation of the content can be prevented. The sealing can also be performed using polyvinylpyrrolidone. Specific examples of sealing methods include a band-sealing method.

The hard capsule of the present invention can be used as an inhalation preparation or a pharmaceutical preparation for rectal administration, in addition to use as a pharmaceutical preparation for oral administration. Further, in addition to drugs for medical treatment, the hard capsule of the present invention can be used in the fields of drugs for animals or plants, cosmetics, and food. Furthermore, reagents or the like used for quantification or synthesis may also be used to fill the capsule of the present invention so as to simplify the procedures therefor.

Examples

Hereinafter, the present invention is described in more detail with reference to Examples and Comparative Examples. However, the present invention is not limited to the following Examples.

In the Examples and Comparative Examples, "%" represents "wt%," unless otherwise specified.

The term "Addition Concentration" in the tables represents a weight (dry weight) of the compound of (B) of the present invention, relative to the total weight of the film. A "size No.3" capsule represents a capsule having a capacity of 0.3 mL, a weight of 0.05 g, a major axis of 1.6 cm, and a minor axis of 0.6 cm.

1. Synthesis of PVA Copolymer

175.8 g of PVA (EG-05; average polymerization degree: 500; saponification degree: 88%; produced by Nippon Synthetic Chemical Industry Co., Ltd.), and 582.3 g of ion exchange water were introduced into a separable flask equipped with a cooling reflux tube, a dropping funnel, a thermometer, a nitrogen inlet tube, and a stirrer. The mixture was dispersed at an ordinary temperature, and then completely dissolved at 95°C. Subsequently, 5.4 g of acrylic acid and 37.3 g of methyl methacrylate were added thereto. After the flask was purged with nitrogen, the temperature was increased to 50°C. Thereafter, 8.5 g of tertiary butyl hydroperoxide and 8.5 g of sodium erythorbate were added thereto, and the reaction was terminated after 4 hours, thereby obtaining a PVA copolymer. The obtained PVA copolymer was dried and pulverized to yield a PVA copolymer powder.

2. Production of Hard Capsule

(1) 3.5 g of sorbitol, 0.35 g of kappa carrageenan, and 0.35 g of potassium chloride were dissolved in 210 g of an aqueous solution containing about a 17% concentration of the copolymer (on a solids basis) obtained through the above-described synthesis, to yield a starting solution for preparing a capsule. The yielded solution was kept warm at about 60°C. A stainless steel pin at room temperature was immersed thereinto and withdrawn to thereby produce a size No. 3 hard capsule having a film thickness of about 0.06 to 0.15 mm. The hard capsule produced in this manner was named "Example A." In Example A, the sorbitol/PVA copolymer (the weight ratio) was about 1/10.

(2) A hard capsule of the same size as that obtained in (1) above was produced in the same manner as in (1) above, except that 3.5 g of mannitol was used in place of 3.5 g of sorbitol. The hard capsule produced in this manner was named "Example B." In Example B, the mannitol/PVA copolymer (the weight ratio) was about 1/10.

(3) Hereinafter, capsules of Examples C to I were produced in the same manner as described above, although the types and amounts of the additives were changed.

Specifically, a hard capsule of the same size as that obtained in (1) above was produced in the same manner as in (1) above, except that 0.35 g of glycerol was used in place of 3.5 g of sorbitol. The hard capsule produced in this manner was named "Example C." In Example C, the glycerol/PVA copolymer (the weight ratio) was about 0.1/10.

Further, a hard capsule of the same size as that obtained in (1) above was produced in the same manner as in (1) above, except that 0.7 g of glycerol was used in place of 3.5 g of sorbitol. The hard capsule produced in this manner was named "Example D." In Example D, the glycerol/PVA copolymer (the weight ratio) was about 0.2/10.

Further, a hard capsule of the same size as that obtained in (1) above was produced in the same manner as in (1) above, except that 1.75 g of glycerol was used in place of 3.5 g of sorbitol. The hard capsule produced in this manner was named "Example E." In Example E, the glycerol/PVA copolymer (the weight ratio) was about 0.5/10.

Further, a hard capsule of the same size as that obtained in (1) above was produced in the same manner as in (1) above, except that 3.5 g of glycerol was used in place of 3.5 g of sorbitol. The hard capsule produced in this manner was named "Example F." In Example F, the glycerol/PVA copolymer (the weight ratio) was about 1/10.

Further, a hard capsule of the same size as that obtained in (1) above was produced in the same manner as in (1) above, except that 0.7 g of triacetin was used in place of 3.5 g of sorbitol. The hard capsule produced in this manner was named "Example G." In Example G, the triacetin/PVA copolymer (the weight ratio) was about 0.2/10.

Further, a hard capsule of the same size as that obtained in (1) above was produced in the same manner as in (1) above, except that 1.75 g of triacetin was used in place of 3.5 g of sorbitol. The hard capsule produced in this manner was named "Example H." In Example H, the triacetin/PVA copolymer (the weight ratio) was about 0.5/10.

Further, a hard capsule of the same size as that obtained in (1) above was produced in the same manner as in (1) above, except that 3.5 g of triacetin was used in place of 3.5 g of sorbitol. The hard capsule produced in this manner was named "Example I." In Example I, the triacetin/PVA copolymer (the weight ratio) was about 1/10.

Further, a hard capsule of the same size as that obtained in (1) above was produced in the same manner as in (1) above, except that 7.0 g of triacetin was used in place of 3.5 g of sorbitol. The hard capsule produced in this manner was named "Example J." In Example J, the triacetin/PVA copolymer (the weight ratio) was about 2/10.

Further, a hard capsule of the same size as that obtained in (1) above was produced in the same manner as in (1) above, except that 1.75 g of triethyl citrate was used in place of 3.5 g of sorbitol. The hard capsule produced in this manner was named "Example K." In Example K, the triethyl citrate/PVA copolymer (the weight ratio) was about 0.5/10.

Further, a hard capsule of the same size as that obtained in (1) above was produced in the same manner as in (1) above, except that 3.5 g of triethyl citrate was used in place of 3.5 g of sorbitol. The hard capsules produced in this manner was named "Example L." In Example L, the triethyl citrate/PVA copolymer (the weight ratio) was about 1/10.

Further, a hard capsule of the same size as that obtained in (1) above was produced in the same manner as in (1) above, except that 1.0 g of propylene glycol was used in place of 3.5 g of sorbitol. The hard capsule produced in this manner was named "Example M." In Example M, the propylene glycol/PVA copolymer (the weight ratio) was about 0.3/10.

Further, a hard capsule of the same size as that obtained in (1) above was produced in the same manner as in (1) above, except that 3.5 g of propylene glycol was used in place of 3.5 g of sorbitol. The hard capsule produced in this manner was named "Example N." In Example N, the propylene glycol/PVA copolymer (the weight ratio) was about 1/10.

(4) For comparison, a gelatin capsule of the same size (size No. 3) of the Japanese Pharmacopoeia Fifteenth Edition was used as "Control A."

Further, a hard capsule of the same size was produced in the same manner as in (1) above, except that 3.5 g of sorbitol was not added. The hard capsule produced in this manner was named "Control B."

3. Evaluation Test

(1) Solubility Test for Hard Capsule

The solubility of the empty hard capsules obtained from the above methods was evaluated in accordance with the process of purity test described in the Item "Capsules" of the Japanese Pharmacopoeia Fifteenth Edition. Specifically, each hard capsule was separated into a cap and a body, and one hard capsule (one pair of a cap and a body) each was separately placed into a 50-mL amount of water having a temperature of $37 \pm 2°C$, which was sometimes stirred. Then, the time taken for complete dissolution was measured. Table 1 shows the results.

Table 1

| Capsule | Compound Name | Addition Concentration | Dissolution Time (minute) |
|---|---|---|---|
| Control A | - | - | 1.5 |
| Control B | None | 0% | 4.8 |
| Example A | Sorbitol | 10% | 4.7 |
| Example B | Mannitol | 10% | 3.9 |
| Example C | Glycerol | 1% | 5.8 |
| Example D | Glycerol | 2% | 5.7 |
| Example E | Glycerol | 5% | 4.3 |
| Example F | Glycerol | 10% | 4.9 |
| Example G | Triacetin | 2% | 3.7 |
| Example H | Triacetin | 5% | 6.2 |
| Example I | Tnacetin | 10% | 4.3 |
| Example J | Triacetin | 20% | 3.1 |
| Example K | Triethyl Citrate | 5% | 5.3 |
| Example L | Triethyl Citrate | 10% | 5.0 |
| Example M | Propylene Glycol | 3% | 6.5 |
| Example N | Propylene Glycol | 10% | 6.7 |

Table 1 confirms that all of the films of the hard capsule of the present invention underwent dissolution within 10 minutes, as defined in the Japanese Pharmacopoeia Fifteenth Edition.

(2) Impact Strength Test of Hard Capsule

Thirty capsules each of Controls A and B, and Examples A to N obtained above were measured for impact strength, after storage in a thermohygrostat for 3 days at 25°C, 40% RH, using an impact strength testing machine (a capsule hardness tester; Qualicaps Co., Ltd.) shown in Fig. 1. Specifically, a 50-g weight was vertically dropped from 5 cm above empty capsules, and the number of damaged capsules was counted. The weight is in the form of a rectangular parallelepiped (height: 4 cm; width: 1.5 cm; and depth: 3 cm). When the cracking of a capsule was confirmed by the naked eye, the capsule was considered to be damaged (broken). Table 2 shows the results.

(3) Moisture Value of Hard Capsule

After the above hard capsules (3 capsules each) were stored for three days at 25°C, 40% RH, they were separated into caps and bodies, and the mass was measured. Then, the resulting products were dried in a dryer at 105°C for 2 hours, and cooled within a desiccator (silica gel) so as to measure the mass again. The moisture value was then calculated from the mass difference before and after drying. Specifically, the difference between the mass before and after drying was considered to be moisture mass, the ratio (%) of the moisture mass in the mass before drying was calculated, and the calculated value was considered to be a moisture value.

Table 2 shows the results.
Table 2

| Capsule | Compound Name | Addition Concentration (%) | Moisture Value (%) | Impact Strength (the number of broken capsutes/the number of sample capsules) |
|---|---|---|---|---|
| Control A | - | - | 12.7 | 0/30 |
| Control B | None | 0 | 4.2 | 30/30 |
| Example | Sorbitol | 10 | 4.0 | 0/30 |
| Example B | Mannitol | 10 | 5.0 | 9/30 |
| Example C | Glycerol | 1 | 4.8 | 11/30 |
| Example D | Glycerol | 2 | 4.1 | 0/30 |
| Example E | Glycerol | 5 | 4.0 | 0/30 |
| Example F | Glycerol | 10 | 5.5 | 0/30 |
| Example G | Triacetin | 2 | 4.1 | 11/30 |
| Example H | Triacetin | 5 | 3.9 | 1/30 |
| Example I | Triacetin | 10 | 3.6 | 0/30 |
| Example J | Triacetin | 20 | 4.6 | 0/30 |
| Example K | Triethyl Citrate | 5 | 3.6 | 7/30 |
| Example L | Triethyl Citrate | 10 | 3.6 | 0/30 |
| Example M | Propylene Glycol | 3 | 4.7 | 5/30 |
| Example N | Propylene Glycol | 10 | 7.5 | 0/30 |

As shown in Table 2, it was confirmed that significantly small numbers of the hard capsules of the present invention were broken, and the hard capsules of the present invention exhibited improved impact strength, compared with the capsules of Control B.

It was also confirmed that an increase in the amount of the compound of (B) of the present invention remarkably improves impact strength.

Further, the moisture values of the hard capsules of the present invention are significantly low, compared with those of gelatin capsules, and are almost equivalent to those of the capsules of Control B. This clarifies that the addition of the compound of (B) of the present invention does not degrade the low moisture property.

(4) Test on Stability of Capsule when Filled with Solvent

0.2 mL each of polyethylene glycol having a weight average molecular weight of 400 (hereinafter referred to as "PEG 400") was used to fill three capsules each of Controls A and B, and Examples A to N, and each of the capsules was band-sealed using a 20% PVA copolymer aqueous solution. Then, the resulting capsules were stored for 7 days at 40°C while stoppered, and changes in appearance, such as a change in the capsule shape, and leakage, as well as the presence of cracking, were confirmed by the naked eye, so as to examine the stability of the capsules when filled with a solvent. The 20% PVA copolymer aqueous solution is an aqueous solution of 20 wt% PVA copolymer powder obtained in "1. Synthesis of PVA Copolymer" above.

The appearance was evaluated in accordance with the following criteria:

I: No change in appearance
II: Slight appearance change, causing no practical problems (no leakage of filled material)
III: Significant appearance change, thus, practically unusable (leakage of filled material)

Table 3

| Capsule | Compound Name | Addition Concentration (%) | Stability when filled with a solvent | |
|---|---|---|---|---|
| | | | Appearance | Broken (the number of broken capsules/the number of sample capsules) |
| Control A | - | - | III | 3/3 |
| Control B | None | 0 | I | 0/3 |
| Example | Sorbitol | 10 | II | 0/3 |
| Example B | Mannitol | 10 | II | 0/3 |
| Example C | Glycerol | 1 | I | 0/3 |
| Example D | Glycerol | 2 | I | 0/3 |
| Example E | Glycerol | 5 | I | 0/3 |
| Example F | Glycerol | 10 | II | 0/3 |
| Example G | Triacetin | 2 | I | 0/3 |
| Example H | Triacetin | 5 | I | 0/3 |
| Example I | Triacetin | 10 | I | 0/3 |
| Example J | Triacetin | 20 | II | 0/3 |
| Example K | Triethyl Citrate | 5 | I | 0/3 |
| Example L | Triethyl Citrate | 10 | II | 0/3 |
| Example M | Propylene Glycol | 3 | I | 0/3 |
| Example N | Propylene Glycol | 10 | I | 0/3 |

Table 3 shows that the gelatin capsules of Control A resulted in noticeable appearance changes and breakage, whereas no change or a slight change was observed in the capsules of the present invention and Control B.

As is also clear from the above results, almost no change in appearance and no cracking were observed even in the capsules of the present invention that were filled with PEG 400. It was thereby confirmed that the capsules of the present invention can be formulated into a pharmaceutical preparation without causing practical problems.

(5) Overall Evaluation

Table 4 summarizes the evaluation test results (1) to (4).
Table 4

| Capsule | Compound Name | Addition Concentration (%) | Solubility Test | Moisture Value (%) | Impact Strength (the number of broken capsules/the number of sample capsules) | Stability when filled with PEG 400 |
|---|---|---|---|---|---|---|
| Control A | - | - | OK | 12.7 | 0/30 | III |
| Control B | None | 0 | OK | 4.2 | 30/30 | I |
| Example A | Sorbitol | 10 | OK | 4.0 | 0/30 | II |
| Example B | Mannitol | 10 | OK | 5.0 | 9/30 | II |

(continued)

| Capsule | Compound Name | Addition Concentration (%) | Solubility Test | Moisture Value (%) | Impact Strength (the number of broken capsules/the number of sample capsules) | Stability when filled with PEG 400 |
|---------|---------------|---------------------------|-----------------|--------------------|------------------------------------------------------------------------------|-----------------------------------|
| Example C | Glycerol | 1 | OK | 4.8 | 11/30 | I |
| Example D | Glycerol | 2 | OK | 4.1 | 0/30 | I |
| Example E | Glycerol | 5 | OK | 4.0 | 0/30 | I |
| Example F | Glycerol | 10 | OK | 5.5 | 0/30 | II |
| Example G | Triacetin | 2 | OK | 4.1 | 11/30 | I |
| Example H | Triacetin | 5 | OK | 3.9 | 1/30 | I |
| Example I | Triacetin | 10 | OK | 3.6 | 0/30 | I |
| Example J | Triacetin | 20 | OK | 4.6 | 0/30 | II |
| Example K | Triethyl Citrate | 5 | OK | 3.6 | 7/30 | I |
| Example L | Triethyl Citrate | 10 | OK | 3.6 | 0/30 | II |
| Example M | Propylene Glycol | 3 | OK | 4.7 | 5/30 | I |
| Example N | Propylene Glycol | 10 | OK | 7.5 | 0/30 | I |

Table 4 shows that the capsules of the present invention have impact resistance that is equivalent to that of known gelatin capsules. Moreover, it was confirmed that the capsules of the present invention exhibit, unlike gelatin capsules, satisfactory physical stability without impairing the low moisture property even when filled with PEG 400.

**Claims**

1. A hard capsule having a film comprising:

   (A) a polymer or copolymer obtained by polymerizing or copolymerizing at least one polymerizable vinyl monomer represented by Formula (1):

   $$H_2C = C(R_1)\text{-}COOR_2 \qquad (1)$$

   wherein $R_1$ represents hydrogen or methyl, and $R_2$ represents hydrogen or alkyl having 1 to 4 carbon atoms, in the presence of polyvinyl alcohol and/or a derivative thereof; and
   (B) at least one compound selected from the group consisting of (B-1) polyhydric alcohols, (B-2) esters of polyhydric alcohols and carboxylic acids having 1 to 5 carbon atoms, and (B-3) esters of polyvalent carboxylic acids and alcohols having 1 to 5 carbon atoms.

2. The hard capsule according to claim 1, wherein, in the compound of (B), the polyhydric alcohol is selected from the group consisting of sorbitol, mannitol, glycerol, and propylene glycol; and the polyvalent carboxylic acid is citric acid.

3. The hard capsule according to claim 1 or 2, wherein the compound of (B) is at least one member selected from the group consisting of glycerol, propylene glycol, triacetin, and triethyl citrate.

4.  The hard capsule according to any of claims 1 to 3, comprising the compound of (B) in an amount of 0.1 to 2 parts by weight, relative to 10 parts by weight of the polymer or copolymer of (A).

5.  The hard capsule according to any of claims 1 to 4, which is to be filled with at least one member selected from the group consisting of

    (a) polyethylene glycols having a weight average molecular weight of 2,000 or less, or derivatives thereof,
    (b) polyoxyethylene sorbitan fatty acid esters,
    (c) fatty acids having 6 to 12 carbon atoms or salts thereof,
    (d) polyoxyethylene castor oil,
    (e) diethylene glycol ether derivatives,
    (f) aliphatic alcohols having 6 to 12 carbon atoms, and
    (g) polyoxyethylene sorbitol fatty acid esters.

6.  The hard capsule according to any of claims 1 to 5, which is filled with at least one member selected from the group consisting of

    (a) polyethylene glycols having a weight average molecular weight of 2,000 or less, or derivatives thereof,
    (b) polyoxyethylene sorbitan fatty acid esters,
    (c) fatty acids having 6 to 12 carbon atoms or salts thereof,
    (d) polyoxyethylene castor oil,
    (e) diethylene glycol ether derivatives,
    (f) aliphatic alcohols having 6 to 12 carbon atoms, and
    (g) polyoxyethylene sorbitol fatty acid esters.

7.  A method for producing a film of the hard capsule of any of claims 1 to 6, comprising:

    immersing a capsule molding pin in an aqueous solution containing (A) a polymer or copolymer obtained by polymerizing or copolymerizing at least one polymerizable vinyl monomer represented by Formula (1):

    $$H_2C = C(R_1)\text{-}COOR_2 \qquad (1)$$

    wherein $R_1$ represents hydrogen or methyl, and $R_2$ represents hydrogen or alkyl having 1 to 4 carbon atoms, in the presence of polyvinyl alcohol and/or a derivative thereof, and (B) at least one compound selected from the group consisting of (B-1) polyhydric alcohols, (B-2) esters of polyhydric alcohols and carboxylic acids having 1 to 5 carbon atoms, and (B-3) esters of polyvalent carboxylic acids and alcohols having 1 to 5 carbon atoms; withdrawing the immersed capsule molding pin from the solution; and drying the aqueous solution adhering to the pin.

8.  Use of (A) a polymer or copolymer obtained by polymerizing or copolymerizing at least one polymerizable vinyl monomer represented by Formula (1):

    $$H_2C = C(R_1)\text{-}COOR_2 \qquad (1)$$

    wherein $R_1$ represents hydrogen or methyl, and $R_2$ represents hydrogen or alkyl having 1 to 4 carbon atoms, in the presence of polyvinyl alcohol and/or a derivative thereof, and (B) at least one compound selected from the group consisting of (B-1) polyhydric alcohols, (B-2) esters of polyhydric alcohols and carboxylic acids having 1 to 5 carbon atoms, and (B-3) esters of polyvalent carboxylic acids and alcohols having 1 to 5 carbon atoms, in producing a hard capsule.

[FIG.1]

Weight 50g

5 cm

Empty Capsule

| **INTERNATIONAL SEARCH REPORT** | International application No. |
|---|---|
| | PCT/JP2010/052091 |

**A.  CLASSIFICATION OF SUBJECT MATTER**
*A61K47/32*(2006.01)i, *A61K9/48*(2006.01)i, *A61K47/08*(2006.01)i, *A61K47/10*
(2006.01)i, *A61K47/12*(2006.01)i, *A61K47/14*(2006.01)i, *A61K47/34*(2006.01)i

According to International Patent Classification (IPC) or to both national classification and IPC

**B.  FIELDS SEARCHED**

Minimum documentation searched (classification system followed by classification symbols)
A61K47/32, A61K9/48, A61K47/08, A61K47/10, A61K47/12, A61K47/14,
A61K47/34

Documentation searched other than minimum documentation to the extent that such documents are included in the fields searched
| Jitsuyo Shinan Koho | 1922-1996 | Jitsuyo Shinan Toroku Koho | 1996-2010 |
|---|---|---|---|
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus/MEDLINE/EMBASE/BIOSIS(STN), JSTPlus/JMEDPlus/JST7580(JDreamII)

**C.  DOCUMENTS CONSIDERED TO BE RELEVANT**

| Category* | Citation of document, with indication, where appropriate, of the relevant passages | Relevant to claim No. |
|---|---|---|
| Y | WO 2002/017848 A1  (Nisshin Kasei Co., Ltd.), 07 March 2002 (07.03.2002), entire text & CN 1449272 A        & EP 1323404 A1 & US 2003/0166763 A1 | 1-8 |
| Y | WO 2005/065716 A1  (Ezaki Glico Co., Ltd.), 21 July 2005 (21.07.2005), paragraph [0041] & JP 2005-194218 A | 1-8 |
| Y | WO 2002/017892 A2  (NOVARTIS NUTRITION AG.), 07 March 2002 (07.03.2002), page 10, paragraph [0003] & EP 1315482 A        & JP 2004-507492 A & US 2003/0165572 A1 | 1-8 |

| ☒   Further documents are listed in the continuation of Box C. | ☐   See patent family annex. |
|---|---|

| *      Special categories of cited documents: | "T"   later document published after the international filing date or priority date and not in conflict with the application but cited to understand the principle or theory underlying the invention |
|---|---|
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 02 March, 2010 (02.03.10) | 09 March, 2010 (09.03.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**EP 2 397 160 A1**

| | INTERNATIONAL SEARCH REPORT | International application No. |
|---|---|---|
| | | PCT/JP2010/052091 |

C (Continuation). DOCUMENTS CONSIDERED TO BE RELEVANT

| Category* | Citation of document, with indication, where appropriate, of the relevant passages | Relevant to claim No. |
|---|---|---|
| Y | JP 8-208458 A (Nippon Elanco Co., Ltd.), 13 August 1996 (13.08.1996), paragraph [0002] (Family: none) | 1-8 |
| Y | JP 11-19503 A (Aicello Chemical Co., Ltd), 26 January 1999 (26.01.1999), claim 2 (Family: none) | 1-8 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2002017848 A

- JP 2552937 B

**Non-patent literature cited in the description**

- *Pharmaceutical Technology Europe,* October 1998, vol. 84 (86), 88-90